# EUROPEAN PATENT APPLICATION

(11) **EP 1 512 328 A1**
(43) Date of publication of application: **09.03.2005**
(21) Application number: 04255312.3
(22) Date of filing: 02.09.2004
(51) Int. Cl.: A23J 3/16, A23J 3/34, A23L 1/305, A23L 1/24, A23L 2/66

(54) **Soluble soy protein with superior functional properties**

(30) Priority: 04.09.2003 US 655158
(71) Applicant: Kraft Foods Holdings, Inc., Northfield, Illinois 60093 (US)
(72) Inventor: Gao, Song, Landsdale Pennsylvania 19446 (US); Smyth, Douglas A., Belvidere New Jersey 07823 (US); Chen, Wen-Shern, Glenview Illinois 60025 (US)
(74) Representative: Smaggasgale, Gillian Helen

(57) **Abstract**

The present invention utilizes a novel enzyme cocktail comprising a fungal protease enzyme or a mixture of fungal protease enzymes having both endo and exo-peptidase activities to hydrolyze soy proteins while substantially avoiding free amino acids and low-molecular weight peptides which impart a bitter or undesirable flavor to the hydrolysate. The hydrolysate, and more preferably the soluble soy protein contained therein, is used in a food product such as, for example, high protein content beverages, sports beverages, balanced nutritional beverages, fruit juice mixes, health/nutrition bars, salad dressings, meat products, snacks, desserts, confectionaries, nutritional supplements, and the like. The soy protein hydrolysate, and more preferably the soluble soy protein contained therein, according to the present invention is particularly useful when the required dose is as high as about 2.5 to about 6.5 grams of soy protein per normal serving of a food product.

## Description

### Field of the Invention

The present invention provides a method using an enzyme (preferably a fungal protease or mixture of fungal proteases) having both endo and exo-peptidase activities to produce a soy protein material having superior functionality (e.g., high solubility and high antioxidant capacity) without the normal bitterness and beany flavor associated with soy materials. The soluble soy protein material of this invention is ideally suited for use in beverages, food products, cosmetics, pharmaceuticals, and the like.

### Background

Soybean rich diets have long been touted to have various health benefits, including serum cholesterol reduction, cancerous or tumor cell inhibition, and immune system stimulation. In addition, the soybean amino acid profile is one of the most complete among vegetable protein sources, and resembles (with the exception of sulfur-containing amino acids) the general patterns derived from high-quality animal protein sources.

On October 26,1999, the FDA accepted scientific evidence that suggests a reduction in the risk of coronary heart disease from soy protein enriched low-fat, low-cholesterol diets, and approved health claims for labeled food products that link intake of at least 6.25 grams of dietary soy protein per reference customarily consumed amount of the food product to a possible reduction in the risk of heart disease. This has intensified efforts to incorporate soy into a wide variety of foods. The benefit of soy protein may be related to its antioxidant activity (see. e.g.,Chen et al., *J. Agric. Food Chem*.,46:49-53 (1998); Chen et al., *J. Agric. Food Chem.,* 43:574-578 (1995); Chen et al., *J*. *Agric. Food Chem.,* 43:574-578 (1996); Suetsuna, *Jpn. Soc. Nutr. Food Sci.,* 52:225-228 (1999); and Zhang et al., *Ann. NY Acad. Sci.*, 864:640-645 (1998)). By scavenging free radicals and oxidative species generated during the course of *in vivo* reactions, the peptides may help protect against pathogenic processes involving enzyme inactivation, DNA mutation, and/or protein denaturation (see, e.g., Szweda et al., *J*. *Biol. Chem.,* 268:3342 (1993); and Reiss et al., *Biochem. Biophys. Res. Commun.,* 48:921 (1972)).

Generally, untreated forms of soy protein are not readily soluble in aqueous liquids, and are difficult to incorporate into various food products, particularly beverages. Soy proteins often have low solubility at pH values of about 6.5 to about 8.5 and often precipitate out at pH values of about 3.5 to about 6.5, thereby imparting a cloudy appearance and/or a sandy texture to the target food product. Untreated soy protein does not generally have significant antioxidant activity although it does contain antioxidant components (e.g., isoflavones) which are associated with or bonded with the soy protein.

Attempts to improve the solubility and other functional properties of soy protein primarily involve hydrolysis. Examples include: U.S. Patent 4,100,024 (using an alkaline proteinase (e.g., *B. licheniformis*) to prepare a soy protein hydrolysate); U.S. Patent 4,478,854 (using a SPS-ase enzyme (e.g., *Aspergillus aculeatus*) to hydrolyze polysaccharides in soy flour decomposed by pectinases); U.S. Patent 4,632,903 (preparing an egg white substitute by enzymatic hydrolysis of soy protein using proteinase (e.g., *Mucor miehei*); reportedly the hydrolysate has superior organoleptic properties); U.S. Patent 5,077,062 (preparing a low sodium, low monosodium glutamate soy hydrolysate by hydrolyzing soy material with a protease enzyme, including fungal proteases derived from *Aspergillus niger*); U.S. Patent Application Publication 2002/132287 (preparing low bitterness hydrolysate through double hydrolysis using *Aspergillus flavus, A. japanicus, A. niger,* or *A. awamori* and papain); U.S. Patents 6,022,702 and 6,126,973 (using a proteolytic enzyme under controlled conditions to selectively decompose β-conglycinin or glycinin to provided soy protein hydrolysate); and copending U.S. Patent Application Serial Number 10/401,131 (filed March 28, 2003 and owned by the same assignee as the present application) (isolating and characterizing peptide antioxidants from protease hydrolyzed soy protein).

Other efforts to incorporate soy protein into foods include: U.S. Patent 5,100,679 (treating a soy protein slurry with a proteolytic enzyme, a carbohydrase enzyme, an antioxidant, or mixtures thereof, followed by treatment with a hydrolyzing agent from a source of alpha-galactosidase for making a proteinaceous product where the alpha-galactosidase can also contain a carbohydrase enzyme and/or a protease enzyme); and U.S. Patent 5,780,439 (providing soy protein hydrolysate by (1) treating a solution of the protein with pepsin, (2) adjust the pH to about 7 to 9, and (3) subjecting the resulting mixture of an enzymatic trypsin-chymostrypsin hydrolysis in the presence of a cationic serine endoprotease).

However, soy protein is known to have an undesirable flavor profile, and attempts to hydrolyze soy protein often produce a bitter hydrolysate. While not bound by any particular theory, it is believed that the bitter taste stems from excess low-molecular fractions and accumulated hydrophobic peptides from the hydrolysis. In the above-referred processes, undesirable hydrolytic fractions were avoided at the price of substantial processing inefficiencies which reduced the degree of hydrolysis (DH, defined as the percent of total peptide bonds cleaved). In other words, the foregoing soy protein hydrolyzing methods avoided low-molecular fractions by early termination of the process, thereby suffering low yields of usable product. For example, in U.S. Patent 4,100,024, a process for reducing bitter tasting low molecular weight polypeptides provides that the DH is ideally 9.5 to 10.5 percent to obtain the best flavor. In another example, U.S. Patent 4,632,903 teaches a hydrolysis of soy proteins to a DH of 0.25 to 2.5 percent to describe the hydrolysis of soy protein for producing an egg-white substitute.

Other efforts to hydrolyze soy protein and prevent bitterness have also been attempted. *See, e*.*g*., Lee et al., "Characterization of hydrolysates produced by mild-acid treatment and enzymatic hydrolysis of defatted soybean flour," *Food Research International*, 34:217 (2001), U.S. Patent 6,537,597, U.S. Patent Application Publication 2002/132287, and U.S. Patent 6,221,423. Lee et al. used prolonged enzymatic hydrolysis of soy protein to form hydrolysates containing short peptides (averaging about 3 to 5 amino acid units) and free amino acids; the degree of hydrolysis (DH) ranged from 20 to 45 percent. U.S. Patent 6,537,597 provided soluble peptides with the degree of hydrolysis generally at 20 to 98 percent (and preferably at 50 to 90 percent). U.S. Patent Application Publication 2002/132287 provided hydrolysate with a degree of hydrolysis ranging from 35 to 45 precent. U.S. Patent 6,221,423 used enzymatic hydrolysis to produce a peptide material with majority of peptides having chain length of 7 amino acid units or less. Although bitterness could be avoided using such prolonged hydrolysis, the health benefits generally associated with soy protein are expected to be significantly reduced or even lost.

Therefore, it would be desirable to provide a soy protein hydrolysis that can increase the soluble fraction, avoid the bitter-tasting fractions (i.e., low molecular weight and hydrophobic peptides), and increase or enhance antioxidant capacity without significantly changing the other health benefits provided by the soy proteins. The present invention generally utilizes a fungal enzyme or cocktail containing at least two fungal enzymes to hydrolyze soy protein to form a soluble soy protein containing relatively large peptides (i.e., an average molecular weight of about 3 to about 30 kDa) without developing off-flavors or bitterness. Thus, a highly-functional soluble soy protein material is prepared which is substantially free of bitter-tasting, low-molecular weight peptides, contains only low amounts of free amino acids, and has a high antioxidant capacity. This highly-functional soluble soy protein can be used to supplement various food products, particularly beverages, dressings, cheese sauces, snacks, desserts, confectionaries, nutritional supplements, and the like. The highly-functional soluble soy protein of the present invention can also be used in other types of products including, for example, cosmetics and pharmaceuticals.

### Summary of the Invention

The present invention provides a method of hydrolyzing soy protein to increase its functionality for food applications (including significantly increased solubility and antioxidant capacity), while avoiding formation of bitter components which are often associated with hydrolyzed soy proteins. The method utilizes an enzyme (preferably a fungal protease enzyme or a mixture of fungal protease enzymes) having both endo and exo-peptidase activities to hydrolyze soy proteins while substantially avoiding the hydrolysis fraction which impart a bitter or undesirable flavor to the finished product. Once the hydrolysis is complete, the enzymes are deactivated by known methods, such as, for example, heating the mixture. Preferably, the resulting soy protein hydrolysate is then separated into a soluble protein fraction and an insoluble or modified protein fraction. The soy protein hydrolysate, the soluble protein fraction, the modified protein fraction, or mixtures thereof can be used in various food products.

The soluble soy protein of the present invention, especially the separated soluble soy protein fraction, has superior functionality. For example, the soluble soy protein of the present invention has substantial antioxidant properties (as measured by oxygen-radical absorbance capacity (ORAC) assay). Thus, the soluble soy protein could be used without other added antioxidants or could be used with added antioxidants to provide even further enhanced antioxidant activity. Though not bound by any theory, the antioxidative properties have been attributed to the generation of antioxidant peptides and/or the presence of isoflavones in the soy protein. The antioxidative property of the soluble soy protein may be used to prevent oxidative damage in food, pharmaceutical, and cosmetic products. In food products, the soluble soy protein can be used to prevent the oxidation of oils and fats, which is associated with loss of nutritious values and causing rancidity. In pharmaceutical and cosmetic products, the soluble soy protein should help to maintain the structural integrity of drugs and skin exposed to destructive oxygen radicals, respectively.

The invention also provides a soluble soy protein with significantly improved solubility (over a wide pH range of about 2 to about 9), bland flavor (i.e., no bitter or off-flavor normally associated with soy beans), and antioxidative activity. This soluble soy protein remains soluble at a low pH of about 2 to about 6. Even at high concentration (i.e., up to about 20 percent), no obvious bitter taste or off-flavor is found. Further, the soluble soy protein may have antioxidative activity that inhibits formation of further off-flavors as well as providing preventive health benefits to consumers.

The present invention also produces food products containing a soluble soy protein material that do not suffer from a cloudy appearance or a sandy texture. Such a product may include, for example, beverages (both neutral and acidic), such as high protein content beverages, sports beverages, balanced nutritional beverages (e.g., 40 percent carbohydrate, 30 percent protein, 30 percent fat), and fruit juice mixes. The soluble soy protein material is also used in other food products, such as health/nutrition bars, salad dressings meat products (e.g., meat spread, sausage, hot dogs, bologna, pepperoni, and the like), snacks, desserts, confectionaries, nutritional supplements, and the like. The soluble soy protein material according to the present invention is particularly useful when the required dose is from about 2.5 to about 6.5 grams of soy protein per normal serving of a food product; of course, lower or higher amounts of soy protein can be used if desired. The soluble soy protein material of the present invention can also be used in other types of products including, for example, cosmetics and pharmaceuticals. The soy protein hydrolysate, the soluble protein fraction, the modified protein fraction, or mixtures thereof can be used in various food and other products.

In one embodiment, the present invention provides a method for preparing a soluble soy protein material, said method comprising:
(1) preparing a hydrolytic mixture comprising water, a soy protein, and an enzyme or a mixture of enzymes having both endo and exo-peptidase activities;
(2) allowing the soy protein to hydrolyze for a sufficient time to produce a soy protein hydrolysate containing at least about 15 percent soluble soy protein; and
(3) deactivating the enzyme or the mixture of enzymes before bitter flavors become noticeable in the soy protein hydrolysate, wherein the soy protein hydrolysate contains the soluble soy protein material. Preferably the enzymes or mixture of enzymes are a fungal protease enzyme or a mixture of fungal protease enzymes. Preferably, the soy protein hydrolysate is separated into a soluble fraction containing the soluble soy protein material and an Insoluble fraction containing an insoluble or modified soy protein and the soluble soy protein material is converted to a dry or powdered form using conventional drying techniques (preferably freeze or spray drying) before use.

In another embodiment, the present invention provides a method for preparing a soluble soy protein material, said method comprising:
(1) mixing a soy protein with water at about 24 to about 55°C to make a soy paste;
(2) adding an enzyme or a mixture of enzymes having both endo and exo-peptidase activities to the soy paste to form a hydrolytic mixture;
(3) incubating the hydrolytic mixture for at least about 30 minutes at a temperature of about 24 to about 55°C to obtain an incubated hydrolytic mixture containing at least about 15 percent soluble soy protein; and
(4) heating the incubated hydrolytic mixture at a temperature of about 80 to about 100°C for at least about 1 minute to inactivate the enzyme or the mixture of enzymes and to obtain a soy protein hydrolysate, wherein the soy protein hydrolysate contains the soluble soy protein material. Preferably the enzymes or mixture of enzymes are a fungal protease enzyme or a mixture of fungal protease enzymes. Preferably, the soy protein hydrolysate is separated into a soluble fraction containing the soluble soy protein material and an insoluble fraction containing an insoluble or modified soy protein and the soluble soy protein material is converted to a dry or powdered form using conventional drying techniques (preferably freeze or spray drying) before use.

In another embodiment, the present invention provides a food product comprising a soluble soy protein material, wherein the soluble soy protein material is prepared by a process comprising:
(1) preparing a hydrolytic mixture comprising a soy protein and an enzyme or a mixture of enzymes having both endo and exo-peptidase activities;
(2) allowing the soy protein to hydrolyze for a sufficient time to produce a soy protein hydrolysate containing at least about 15 percent of the soluble soy protein; and
(3) deactivating the enzyme or the mixture of enzymes blend before bitter flavors become noticeable in the soy protein hydrolysate. Preferably the enzymes or mixture of enzymes are a fungal protease enzyme or a mixture of fungal protease enzymes. Preferably, the soy protein hydrolysate is separated into a soluble fraction containing the soluble soy protein material and an insoluble fraction containing an insoluble or modified soy protein.
Even more preferably, the separated soluble soy, protein material is converted to a dry or powdered form using conventional drying techniques (preferably freeze or spray drying) before use.

In another embodiment, the present invention provides a method for preparing a soluble soy protein material, said method comprising:
(1) mixing a soy protein-containing material with water at about 24 to about 55°C to make a soy paste containing about 10 to about 20 percent soy protein at a pH of about 6.5 to about 8.0;
(2) adding about 0.01 to about 0.5 percent of an enzyme or a mixture of enzymes (preferably a fungal protease enzyme or a mixture of fungal protease enzymes) having both endo and exo-peptidase activities to the soy paste to form a hydrolytic mixture;
(3) incubating the hydrolytic mixture for about 0.5 to about 5 hours at a temperature of about 24 to about 55°C to obtain an incubated hydrolytic mixture containing at least about 15 percent soluble soy protein; and
(4) heating the incubated hydrolytic mixture at a temperature of about 80 to about 100°C for about 10 sec to about 25 minutes to deactivate the enzyme or the mixture of enzymes and to obtain a soy protein hydrolysate, wherein the soy protein hydrolysate contains the soluble soy protein material;
(5) adjusting the pH of the soy protein hydrolysate to about 3.5 to about 5.5;
(6) treating the pH-adjusted soy protein hydrolysate to separate the soluble soy protein from insoluble/modified soy protein, wherein the soluble soy protein is obtained in an amount of at least about 15 to about 45 percent of the soy protein-containing material; and
(7) drying the separated soluble soy protein to obtain the soluble soy protein in a solid or powdered form.

The soluble soy protein soy materials of the present invention are ideally suited for use in dairy and non-dairy beverages, smoothies, health drinks, confectionary type products, nutritional bars, cheeses, cheese analogs, dairy and non-dairy yogurts, meat and meat analog products, cereals, baked products, snacks, health/nutrition bars, confectionaries, nutritional supplements, salad dressings, meat products (e.g., meat spread, sausage, hot dogs, bologna, pepperoni, and the like). The soluble soy protein material according to the present invention is particularly useful when the required dose is from about 2.5 to about 6.5 grams of soy protein per normal serving of a food product; of course, lower or higher amounts of soy protein can be used if desired. The soluble soy protein material of the present invention can also be used in other types of products including, for example, cosmetics and pharmaceuticals.

### Detailed Description

The hydrolysis is carried out using an enzyme or mixture of enzymes, preferably a fungal protease enzyme or a mixture of fungal protease enzymes, having both endo and exo-peptidase activities to hydrolyze soy proteins while substantially avoiding the hydrolysis fraction which impart a bitter or undesirable flavor to the finished product. This class of enzymes has been found to hydrolyze soy proteins without releasing significant levels of low molecular weight soy protein peptides (i.e., molecular weights less than about 3000 Daltons and preferably less than about 2000 Daltons) or free amino acids which may impart bitter taste to the hydrolysate. Generally, the hydrolysate produced by this invention contains at least about 15 percent, and preferably about 20 to about 45 percent, soluble soy protein and is substantially free of low molecular weight soy protein peptides. For purposes of this invention, "substantially free of low molecular weight protein peptides" is a level such that a bitter taste is not developed in the resulting hydrolysate. Generally, such substantially free of low molecular weight soy protein hydrolysate contains less than about 5 percent of low molecular weight peptides (i.e., having molecular weight less than about 3000 Daltons) and less than about 5 percent, preferably less than about 3 percent, and more preferably less than about 1 percent, free amino acids. Such substantially free of low molecular weight soy protein hydrolysate is preferably further processed to separate the soluble soy protein and insoluble or modified soy protein using conventional techniques (e.g., centrifugation, filtration, and the like). The separated soluble soy protein is soluble in and forms a substantially clear solution in water or acidic solutions. The separated soluble soy protein fraction is also substantially free of low molecular weigh peptides (i.e., generally less than about 10 percent of peptides having molecular wieghts less than about 3000 Daltons) and free amino acids (generally less than about 7.5 percent). Protein solubility can be determined as described in Franzen et al., *J*. *Agric. Food Chem.,* 24, 788795 (1976), which is hereby incorporated by reference.

The soy protein used in the present invention may be taken from the group consisting of soy protein isolate, soy protein concentrate, soy protein extract, soy flour, powdered or dry soy milk, soy meal, ground soy bean, soy bean paste, and mixtures thereof. The soy protein used in the present invention may also, if desired, be deflavored, prior to the hydrolysis of the present invention, using the procedures outlined in copending United States Patent Application Serial Numbers 09/939,500, filed August 23, 2001, and 10/655,259, filed on the same date as the present application, both of which are incorporated by reference. Generally, soy protein isolates having a protein content of about 80 to about 94 percent, and more preferably about 85 to about 90 percent, are preferred in the present invention.

In one embodiment, the method is carried out by: (1) preparing a hydrolytic mixture comprising water, a soy protein, and an enzyme or mixture of enzymes (preferably a fungal protease enzyme or a mixture of fungal protease enzymes) having both endo and exo-peptidase activities; (2) allowing the soy protein to hydrolyze for a sufficient time to produce a soy protein hydrolysate containing at least about 15 percent soluble soy protein; and (3) deactivating the enzyme or a mixture of enzymes before bitter flavors become noticeable in the soy protein hydrolysate, wherein the soy protein hydrolysate contains the soluble soy protein material. Preferably the soluble soy protein material is separated from the soy protein hydrolysate and then obtained in a solid or powdered form.

in another embodiment, the method is carried out by: (1) mixing a soy protein with water at about 24 to about 55°C to make a soy paste; (2) adding a enzyme or a mixture of enzymes (preferably a fungal protease enzyme or a mixture of fungal protease enzymes) having both endo and exo-peptidase activities to the soy paste to form a hydrolytic mixture; (3) incubating the hydrolytic mixture for at least about 30 minutes at a temperature of about 24 to about 55°C to obtain an incubated hydrolytic mixture containing at least about 15 percent soluble soy protein; and (4) heating the incubated hydrolytic mixture at a temperature of about 80 to about 100°C for at least about 1 minute to inactivate the enzyme or the mixture of enzymes and to obtain the soy protein hydrolysate, wherein the soy protein hydrolysate contains the soluble soy protein material. Preferably the soluble soy protein material is separated from the soy protein hydrolysate and then obtained in a solid or powdered form.

The final hydrolyzed soy product contains an insoluble fraction in solid form and a soluble fraction which remains solubilized in the supernatant solution. The soluble and insoluble fractions can be separated by known methods, such as, for example, by centrifuge. Generally, the insoluble fraction will have a higher average molecular weight than the soluble fraction. Once separated, the solution containing the low molecular weight fraction or soluble soy protein fraction can be utilized in food applications as is, or, more preferably further processed into a solid or powdered form for use in food applications. Generally, the soluble soy protein fraction is substantially free of low molecular weight soy proteins (i.e., it contains less than about 10 percent of low molecular weight peptides (i.e., having molecular weight less than about 3000 Daltons) and less than about 7.5 percent free amino acids). Generally, the soluble soy protein fraction comprises peptides having an average molecular weight of about 3 to about 30 kDa. Generally, the soluble soy protein fraction is soluble in an aqueous medium having a pH of about 2 to about 9.

The insoluble soy protein fraction contains insoluble or modified soy proteins. This fraction can also be used in food products, especially in semisolid or solid food products such as pasta, cereal, and the like. The insoluble soy protein fraction, especially when prepared from deflavored soy materials such as soy flour, can provide a good source of soy protein and fiber.

The enzymes or mixture of enzymes used in the present invention have both endo- and exo-peptidase activities. Preferably the enzymes used in the present invention comprise a fungal protease enzyme or a mixture of fungal protease enzymes having both endo- and exo-peptidase activities. Such fungal protease enzymes are commercially available. Examples of suitable fungal protease enzymes include, for example, Corolase PN-L (AS Enzymes, Finland; a fungal proteinase produced from *Aspergillus sojae* with high levels of endo- and exo-peptidase activities); Flavorurzyme 500L (Novozymes North America Inc., Franklinton, N.C; a fungal protease/peptidase complex produced from *Aspergillus oryzae* and which contains both endoprotease and exopeptidase activities); Fungal Protease 500,000 and Fungal Protease Concentrate (Genencor International, Rochester, NY; *Aspergillus oryzae* fungal protease preparations with both endo and exo-peptidase activities).

The invention is further described by the examples below. It should be recognized that variations based on the inventive features disclosed herein are within the skill of the ordinary artisan, and that the scope of the invention should not be limited by the examples. To properly determine the scope of the invention, an interested party should consider the claims herein, and any equivalent thereof. In addition, all citations herein are incorporated by reference, and unless otherwise expressly stated, all percentages and ratios are by weight.

**Example 1. Screening of enzymes to give non-bitter soy protein hydrolysate**. The hydrolysis of soy proteins using protease enzymes usually generates bitter taste due to the release of bitter peptides during enzyme digestion. The following protease enzymes were selected for evaluation:

| | |
|---|---|
| Valley Research | Validase TSP Concentrate II |
| AB Enzyme | Corolase 7089 |
| | Corolase PN-L |
| Novozyme | Flavourzyme 500L |
| | Alcalase 2.4L FG |
| | Protamex |
| | Neutrase 1.5 MG |
| Genencor | Fungal Protease 500,000 |
| | Protex 6L |
| | Multifect Neutral |
| | Fungal Protease Concentrate |

For evaluation, a soy protein isolate (TX34; Protein Technologies lnternation, St. Louis, MO) in a water suspension (15%) was treated with 1 percent of the enzymes listed above at 50°C (except that the Protamex enzyme treatment was at 38°C) for about 30 to about 90 minutes. The enzymes were then inactivated in boiling water for 6-7 minutes. The resulting hydrolysates were then evaluated using an informal taste panel (8 or 9 members) using a 0 (not bitter) to 10 (extremely bitter) scale. Some samples were so bitter that they were not evaluated by the taste panel; these samples are identified as "very bitter" in the table below. A control sample was prepared using the same procedure except that no enzyme was added. A score of 4 or less was considered acceptable. The following results were obtained:

| **Enzyme** | **Taste Score** |
|---|---|
| Control | 2.2 |
| Validase TSP Concentrate II | 9.6 |
| Corolase 7089 | very bitter |
| Corolase PN-L | not bitter* |
| Flavourzyme SOOL | 3.4 |
| Alcalase 2.4L FG | very bitter |
| Protamex | 9.6 |
| Neutrase 1.5 MG | very bitter |
| Fungal Protease 500,000 | 1.3 |
| Protex 6L | very bitter |
| Multifect Neutral | very bitter |
| Fungal Protease Concentrate | 3.5 |

| | |
|---|---|
| * Material was not evaluated by the taste panel to provide a numerical bitterness result. it was, however, not bitter and would be expected to have a value lower than 4. | |

Amount the enzymes tester here, only Flavourzyme 500L, Fungal Protease Concentrate, Fungal Protease 500,000, and Coralase PN-L provided soy hydrolysates that were acceptable in the taste test. Flavourryme 500 L, Fungal Protease Concentrate, Fungal Protease 500,000 are endo/exo-peptidase complexes produced from the fungus strain *Aspergillus oryzae*; Corolase PN-L is an endo/exo-peptidase complex from *Aspergillus sojae.*

**Example 2**. The acceptable enzymes from Example 1 were used to treat a variety of soy protein materials. The soy protein materials tested were as follows: (1) Soy protein isolate TX34 from Dupont Protein Technologies; and (2) deflavored soy flour (DFSF; 65 percent protein). Several mixtures of enzymes were used: (1) 1:1 blend of Fungal Protease Concentrate (FPC) and Fungal Protease 500,000 (FP500); and (2) 1:1 blend of Flavourzyme 500L (Flav) and Fungal Protease Concentrate (FPC).

A slurry of about 200 g of the soy protein material in about 1 to about 1.5 L water was preheated to about 50°C in a water bath or jacketed container. About 1 to about 3 g of one or more of the fungal protease enzymes was then added to the slurry. The resulting mixture was then gently stirred for about 30 to about 90 minutes at about 50°C. The mixture was then heated in a boiling water bath for about 5 to about 20 minutes in order to inactivate the enzyme; generally a temperature of about 80 to about 95°C is sufficient for inactivation. After cooling to ambient temperature, the mixture was centrifuged at about 13,500 to about 22,000 G for about 5 to about 15 minutes. If desired, the pH of the mixture could be adjusted to about 4.5 by the addition of an edible acid (e.g., lactic acid, citric acid, phosphoric acid, and the like as well as mixtures thereof) prior to centrifugation; without acid addition, the pH of the mixture was about 6.2 to about 6.8. Generally, if the soluble soy protein is to be used in high acid products (e.g., fruit juices), it is preferred to adjust the pH to about 3.5 to about 5.5; for use in products such as cheese sauces, it is generally preferred that the pH not be adjusted at this stage.

The supernatant containing soluble soy protein was separated from the solid pellet. The supernatant was then freeze dried to obtain the soluble soy protein in dried form. Alternatively, the supernatant could be spray dried. The yield of dried soluble soy protein was about 25 to about 45 percent and was generally independent of whether or not the pH was adjusted prior to centrifugation. The soluble soy protein powder (SolSP) can also designated as low molecular weight fraction (LMWF or LMW fraction), If desired, this may be centrifuged and/or filtered again. The pellet was dispersed in 1 to 2 volume of water (if the pH had been adjusted previously, the pH of the aqueous dispersion was adjusted to about 6.8 to about 7.2 with 1N to 6N NaOH) and then freeze-dried (or spray dried) to obtain modified soy protein (MSP; also designated as the high molecular weight fraction (HMWF or HMW fraction)).

| | Soy Protein | | | | | |
|---|---|---|---|---|---|---|
| No. | ID | Amount (g) | Enzyme | Reaction Time (min) | Soluble Soy Protein (%) | Hydrolysate (%) |
| 1 | TX34 | 100 | 0.5 g FPC / 0.5 g FP500 | 57 | 41.2 | - |
| 2* | TX34 | 150 | 0.72 g FPC / 0.72 g FP500 | 57 | 40.3 | - |
| 3** | TX34 | 150 | 0.72 g FPC / 0.72 g FP500 | 52 | - | 98 |
| 4 | DFS F | 160 | 0.5 Flav / 0.5 FPC | 120 | 20.0 | - |
| 5^{†} | DFS F | - 160 | 0.5 Flav / 0.5 FPC | 60 | 20.4 | - |

| | | | | | | |
|---|---|---|---|---|---|---|
| * Soluble soy protein was obtained at pH 4.5 | | | | | | |
| ** Hydrolystate obtained without centrifugation or adjusting pH. | | | | | | |
| ^{†} pH of starting slurry was adjusted to 7.4. | | | | | | |

The amino acid profiles and the amount of free amino acids in the starting material and the products of the above table were determined and were as follows:

| Sample | Starting TX34 | 1 | 2 | 3 | Starting 4 5 DFSF | | |
|---|---|---|---|---|---|---|---|
| | | | | | | | |
| Protein (%) | 83.3 | 90.9 | 75.2 | 83.6 | 65.5 | 77.6 | 78.2 |
| | | | | | | | |

| Amino acid profile (%) | | | | | | | |
|---|---|---|---|---|---|---|---|
| Asp | 10.0 | 9.7 | 8.2 | 9.5 | 7.7 | 7.6 | 7.03 |
| Thr | 2.7 | 2.1 | 1.9 | 2.5 | 2.2 | 2.8 | 2.7 |
| Ser | 4.0 | 4.8 | 4.0 | 4.6 | 4.0 | 4.1 | 4.1 |
| Glu | 16.1 | 18.2 | 16.4 | 15.3 | 12.4 | 13.3 | 11.9 |
| Gly | 3.1 | 3.2 | 2.8 | 3.5 | 2.9 | 3.0 | 2.9 |
| Ala | 3.8 | 2.6 | 2.5 | 3.3 | 2.8 | 3.2 | 3.2 |
| Val | 4.5 | 3.0 | 2.9 | 4.3 | 3.3 | 3.0 | 3.1 |
| Met | 1.1 | 0.9 | 0.8 | 1.1 | 1.0 | 1.1 | 1.1 |
| Ile | 4.0 | 3.0 | 2.8 | 4.2 | 3.2 | 2.7 | 2.8 |
| Leu | 6.9 | 5.3 | 4.6 | 6.9 | 5.4 | 4.2 | 4.4 |
| Tyr | 2.9 | 2.2 | 2.0 | 3.2 | 2.5 | 2.0 | 2.3 |
| Phe | 4.4 | 3.8 | 3.2 | 4.6 | 3.5 | 2.5 | 2.8 |
| Lys | 4.3 | 5.8 | 5.3 | 5.4 | 4.2 | 5.9 | 5.6 |
| His | 1.7 | 2.1 | 1.8 | 2.1 | 1.8 | 2.2 | 2.2 |
| Arg | 5.0 | 6.4 | 6.0 | 6.2 | 4.6 | 4.1 | 4.5 |
| Pro | 4.0 | 5.3 | 4.2 | 5.0 | 4.0 | 4.4 | 4.4 |
| Cys | 0.8 | 0.8 | 0.7 | 0.9 | 0.8 | 1.0 | 1.0 |
| Trp | 0.9 | 0.6 | 0.4 | 0.9 | 0.3 | 0.6 | 0.6 |
| Total (%) | 79.9 | 79.8 | 70.6 | 83.5 | 66.7 | 67.7 | 66.4 |
| | | | | | | | |

| Free Amino acid in fractions (%) | | | | | | | |
|---|---|---|---|---|---|---|---|
| Asp | <0.01 | 0.03 | 0.02 | 0.03 | 0.01 | 0.4 | 0.33 |
| Thr | <0.01 | 0.57 | 0.37 | 0.57 | 0.03 | 1.17 | 1.23 |
| Ser | <0.01 | <0.01 | <0.01 | <0.01 | <0.01 | <0.01 | <0.01 |
| Glu | <0.01 | 0.09 | 0.04 | 0.05 | 0.02 | 0.75 | 0.6 |
| Gly | <0.01 | 0.01 | 0.01 | 0.02 | 0.01 | 0.08 | 0.11 |
| Ala | <0.01 | 0.14 | 0.11 | 0.1 | 0.04 | 0.52 | 0.49 |
| Val | <0.01 | 0.14 | 0.05 | 0.09 | 0.01 | 0.33 | 0.42 |
| Met | <0.01 | 0.07 | 0.06 | 0.05 | <0.01 | 0.19 | 0.18 |
| Ile | <0.01 | 0.16 | 0.09 | 0.11 | <0.01 | 0.27 | 0.36 |
| Leu | <0.01 | 0.9 | 0.42 | 0.49 | 0.01 | 0.93 | 0.97 |
| Tyr | <0.01 | 0.16 | 0.1 | 0.1 | 0.01 | 0.09 | 0.37 |
| Phe | <0.01 | 0.85 | 0.41 | 0.47 | 0.01 | 0.63 | 0.86 |
| Lys | <0.01 | 0.75 | 0.5 | 0.31 | 0.06 | 1.33 | 0.95 |
| His | <0.01 | 0.1 | 0.07 | 0.06 | 0.01 | 0.21 | 0.25 |
| Arg | <0.01 | 0.81 | 0.48 | 0.32 | 0.01 | 0.1 | 0.1 |
| Pro | <0.01 | 0.04 | <0.01 | <0.01 | 0.02 | 0.06 | 0.02 |
| Total (%) | 0 | 4.8 | 2.7 | 2.8 | 0.25 | 7.1 | 7.2 |

Samples 1, 2, and 3 were SoISP obtained from the TX34 starting material and samples 4 and 5 were SoISP obtained form the DFSF starting material. The soluble soy protein samples have protein levels of between about 75 and about 91 percent and contain a good balance of all essential amino acids and maintain a good balance. The soluble soy protein samples contained less than about 7.5 percent free amino acids.

Other soy protein materials were hydrolyzed as above. These soy materials included (1) two deflavored soy flour (DFSF and DFSF2 containing 65 and 70 percent protein respectively); (2) deflavored soy extract (DFSE containing 90 percent protein): (3) soy protein isolate from Archer Midland Daniels (ADM 974 containing 90 percent protein); and (4) soy protein isolate from Protein Technologies International (TX34 containing 83 percent protein). The enzyme. used was fungal protease concentrate alone or in combination with Flavorzyme; generally such blends contained fungal protease concentrate and Flavorzyme in a ratio of about 1:5 to about 5:1. The results are as follows:

| Soy protein | | Soluble Soy Protein (SolSP) | | | Modified Soy Protein (MSP) | |
|---|---|---|---|---|---|---|
| ID | Starting Amount (g) | Amount (g) | Protein (%) | Protein yield (%) | Amount (g) | Protein (%) |
| SF2 | 150 | 35.5 | 86.2 | 29.3 | na | na |
| DFSE | 120 | 34.7 | 96.6 | 31.4 | na | na |
| DFSF2 | 150 | 39.7 | 70.4 | 26.8 | na | na |
| DFSE | 120 | 37.8* | 78.7 | 27.9 | na | na |
| ADM 974 | 200 | 84.0 | 85.1 | 39.7 | 119.3 | 83.2 |
| TX34 | 200 | 84.7 | 81.3 | 41.3 | 100 | 71.7 |
| TX34 | 200 | 86.4 | 81.7 | 42.4 | 119 | 78.5 |
| TX34 | 200 | 78.4 | 78.38 | 36.9 | 126.5 | 77.4 |
| DFSF2 | 250 | 70.0 | 67.6 | 27.2 | 195.5 | 86 |
| DFSE | 200 | 60.6* | 72.2 | na | 96 | na |

| | | | | | | |
|---|---|---|---|---|---|---|
| * Portion lost during processing; "na" = not applicable. | | | | | | |

Gel electrophoresis was used to analyze the protein profiles (size distribution) of the various starting materials and soluble proteins obtained through hydrolysis from the previous table. The following results were obtained:

| | Protein Fractions (%) | | | |
|---|---|---|---|---|
| SAMPLE* | >27 KDa | 14-27 KDa | 3.5-14 KDa | <3.5 KDa |
| Soy flour | 69.8 | 26.1 | 4.1 | nd |
| Soy flour-2 | 72.2 | 24.5 | 3.3 | nd |
| SoISP-DFSF2 | 33.5 | 53.6 | 12.9 | nd |
| SolSP-DFSE | 10.2 | 52.1 | 37.7 | nd |
| SolSP-DFSF2-3 (pH 4.5) | 10.1 | 59.3 | 30.6 | nd |
| SoISP-DFSE-3 (pH 4.5) | 11.9 | 48.6 | 39.5 | nd |
| SolSP-ADM 974 (pH 4.5) | 8.8 | 35.6 | 55.6 | nd |
| SolSP-TX34 (pH 4.5) | 12.6 | 48.5 | 38.9 | nd |
| SolSP-TX34-2 (pH 4.5) | 12.7 | 53.1 | 35.2 | nd |
| SolSP-DFSF2-2** (pH 4.5) | 16.2 | 37.9 | 45.9 | nd |
| SolSP-DFSE-2*** (pH 4.5) | 19.6 | 30.0 | 50.4 | nd |
| MSP-DFSF2 | 51.5 | 26.7 | 21.8 | nd |
| MSP-DFSE | 54.5 | 32.6 | 12.9 | nd |
| MSP-DFSF2-3 | 61.8 | 19.1 | 19.1 | nd |
| MSP-ADM974 | 18.6 | 30.6 | 50.8 | nd |
| MSP-TX34 | 29.0 | 28.9 | 42.1 | nd |
| MSP-DFSE-2 | 62.0 | 18.2 | 19.8 | nd |

| | | | | |
|---|---|---|---|---|
| * SolSP = soluble soy protein from hydrolysis; MSP = modified soy protein from hydrolysis. | | | | |
| ** The hydrolysis was started at pH 7.2. | | | | |
| *** The hydrolysis was started at pH 7.6. nd = Not detected by gel electrophoresis. | | | | |

Hydrolysis decreased the amount of higher molecular proteins while significantly increasing the amount of lower molecular weight proteins (i.e., proteins in the ranges of 3.5 to 14 KDa and 14 to 27 KDa). The starting soy flour had about 70 percent protein with molecular weight >27 KDa. After the enzyme treatment and processing, about 80 to about 90 percent of the soluble soy proteins (except one sample) fall in within 3.5 to 14 KDa and 14 to 27 KDa molecular weight ranges with about 30 to about 60 percent in the 14 to 27 KDa range.

These results clearly demonstrate that the enzyme treatment of the present invention provides soluble soy protein compositions containing mainly large proteins or peptides with a relatively small amount of free amino acids (generally less than about 10 percent).

The soluble soy protein is ideally suited for use in food products, especially beverages, in which a clear appearance is important. The modified soy protein can be used as a soy protein isolate and included in food products such as, for example, nutritional bars, pasta, process cheese, cereal, and the like.

This enzymatic hydrolysis could also be applied on other general sources of protein, such as casein and whey protein, to improve functionality and flavor without generating the bitter taste peptides.

**Example 3**. This example illustrates the high antioxidant capacity of soluble soy proteins prepared in this invention. Batches of soluble soy proteins produced in Example 2 were evaluated by ORAC analysis. ORAC reflects antioxidant capacity and provides a measure of scavenging capacity of antioxidants against the peroxyl radical. Among different reactive oxygen species, peroxyl radical is one of the most reactive and common radicals found in body (see, e.g., Cao et. al. *Free Radical Biology & Medicine,* 1993, 303-11; Wang et al., *J*. *Agric. Food Chem.,* 1996, 44, 701-5).

ORAC results of the soluble soy proteins prepared in this invention are provided in the table below along with ORAC results reported in the literature for a number of fruit samples (Wang et al., *J. Agric. Food Chem*., 44, 701-705 (1996)). Such fruits are generally considered to be high in antioxidative properties.

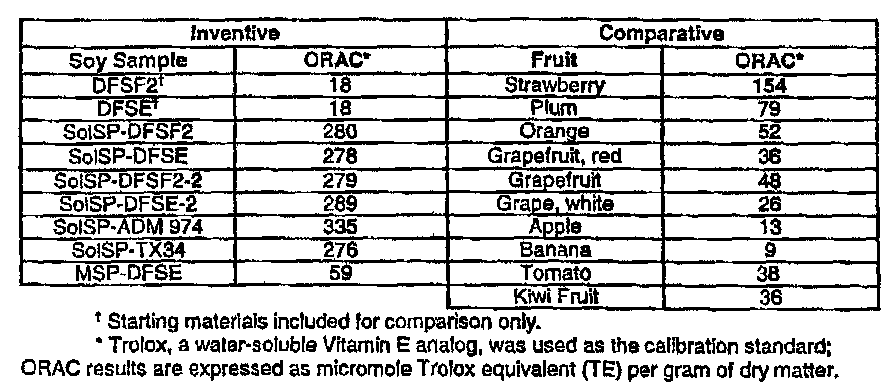

These soluble soy proteins prepared in this invention have exceptionally high antioxidant capacity. The ORAC values of soluble soy protein increased over 15 folds compare to the soy starting material. They are also significantly higher than fruits. Therefore, the soluble soy proteins of this invention, with about 10 times of antioxidant capacity of grape on dry basis, are especially useful for providing an antioxidant beneficial to human health and for preventing rancidity or oxidation in food or beverage products that contain unsaturated fatty acids or oil to improve product shelf life and quality. The modified soy proteins (MSP) also have elevated antioxidant capacities which are comparable to fruits. These compositions (i.e., the soluble proteins and the modified proteins) may be used in food products, pharmaceutical products, nutritional supplements, and cosmetic products.

**Example 4**. This example illustrates the use of the soluble soy protein of this invention in Tang® type beverage. Samples were prepared by mixing about 8.3 g (75% soy protein) of the various soluble soy proteins (i.e., produced from soy protein isolate TX34, soy protein isolate ADM 974, DFSE, and DFSF) in about 230 ml water and then adding about 25 g Tang® orange flavor powder; the mixture was stirred until dissolved. Samples were evaluated using a six-member taste panel and a score of 0 (not bitter) to 1.0 (extremely bitter) and compared against a control (25 g Tang® orange flavor powder in 230 ml water). The following results were obtained:

| Sample | Bitterness |
|---|---|
| Control | 1.2 |
| SolSP-TX34 (pH 4.5) | 4.4 |
| SolSP-ADM974 (pH 4.5) | 2.9 |
| SolSP-TX34 (pH 4.5) | 4.0 |
| SolSP-DFSF2 (pH 4.5) | 1.5 |
| SolSP-DFSE (pH 4.5) | 2.0 |
| SoISP-DFSF2 (pH 4.5) | 1.8 |

Of the six tested samples, four samples were not considered bitter (score less than 4). The two other samples were only considered as slightly bitter with some panelists not finding them bitter at all.

The inclusion of the soluble soy protein at levels of about 8 to about 15 g soluble soy protein in 230 ml water (with 25 g Tang® powder) did not significantly effect viscosity (as compared to control sample). Moreover, the inclusion of such levels of soluble protein did not adversely effect appearance, texture, or color while providing significant levels of soy protein.

**Example 5**. This example illustrates the use of the soluble soy protein of this invention in fruit beverages. Soluble soy protein (5.5 g; 85 percent protein; prepared from ADM 974 as in Example 2) was dissolved in 6 oz Tropicana® grapefruit juice or in 6 oz Tropicana® grapefruit juice enriched with calcium. Another soluble soy protein (5.8 g; 78 percent protein; prepared from DFSE as in Example 2) was dissolved in 6 oz Tropicana® premium 100% squeezed orange juice. All juices enriched with the soluble soy protein of this invention were similar to control samples in taste, texture, and appearance.

**Example 6**. This example illustrates the use of the soluble soy protein of this invention in cheese products. Soluble soy protein (7.0 g; 88 percent protein; prepared from DFSE similar to that in Example 2) was added to the cheese sauce (about 21-22 g) used in Kraft Easy Mac®. The soy supplemented sauce was mixed with cooked macaroni (when hot or immediately after taking out from a microwave oven after 4-minute cooking). The texture and flavor of the soy supplemented product was similar to a control sample (no added soy protein).

**Example 7**. This example illustrates the use of the soluble soy protein of this invention in caramel. Caramel is widely used in many snack and confectionary products. Thus, caramel could be used as a vehicle to deliver soy protein into various products.

In a first sample, soluble soy protein (3.0 g; 96.6 percent protein; prepared from DFSE as in Example 2) was dissolved in 14 g of a first melted commercially available caramel. In a second sample, the same soluble soy protein (1.05g; 96.6 percent protein) was dissolved in 11.6 g of a second melted commercially available caramel, The caramel samples were cooled to room temperature and evaluated. The soy enriched caramels had similar taste to the control caramels (no added soy).

**Example 8**. This example illustrates the use of the soluble soy protein of this invention in dressing products. Soluble soy protein (5.0 g; 85 percent protein; prepared from ADM 974 as in Example 2) was dissolved in 20.7 g of a commercially available dressing (Kraft® Ranch Dressing). The resulting product has similar rheology and flavor as a control sample.

**Example 9.** This example illustrates the effect of hydrolysis conditions in the process of this invention. Generally, it was found that both the yield and the protein content (i.e., purity) of the resulting soluble soy protein increases as the starting material's protein content increases and/or incubation time increases.

Deflavored soy flour (DFSF; 250 g; 65 percent protein) was dispersed in sufficient water to obtain a 15.6 percent slurry. The pH was adjusted to 7.5 with 1 N NaOH at room temperature (RT); after increasing the temperature to 50°C, about 0.5 percent of an enzyme blend (i.e., 3 parts Fungal Protease Concentrate and 1 part Corolase PN-L), based on protein weight, was added. The mixture was agitated and incubated at 50°C for 3 hr. The enzymes were inactivated by heating in a boiling water bath for about 10-12 minutes. After cooling to room temperature, the pH of the mixture was adjusted to about 4.5 by adding lactic acid. The acidified mixture was centrifuged under conditions to provide a clear supernatant and a pellet. The supernatant was collected and freeze dried to obtain about 71 g soluble soy protein (about 28.4 percent yield with about 64 percent protein).

This experiment was repeated with different levels of enzymes and incubation times. The following results were obtained:

| Enzyme (%) | Incubation Time (hr) | Yield (%) | Protein (%) |
|---|---|---|---|
| 1.0 | 2.5 | 31.2 | 64.8 |
| 0.75 | 2.5 | 26.3 | 62.2 |
| 0.5 | 2.5 | 27.6 | 61.1 |
| 0.5 | 3.0 | 28.4 | 64.0 |
| 0.25 | 2.5 | 25.2 | 58.2 |
| 0.1 | 3.0 | 21.2 | 53.0 |

In another experiment, deflavored soy protein extract (DFSE; 64 g, 89 percent protein) was dispersed in sufficient water to obtain a 14 percent slurry. The pH was adjusted to about 7.6 by adding 2N NaOH at room temperature. After heating to 50°C in a water bath, 0.5 percent of an enzyme blend (3 parts Fungal Protease Concentrate and 1 parts Corolase PN-L), based on protein weight, was added to the dispersion. Hydrolysis was continued for 2.5 hours at 50°C. The enzymes were then inactivated in a boiling water bath for about 10 minutes. After cooling to room temperature, the pH of the mixture was adjusted to 4.5 by lactic acid (85 percent) and citric acid (15 percent), The acidified mixture was centrifuged to obtain the supernatant. After freeze-drying the supernatant, 23.6 g of soluble soy protein (36.9 percent yield with 73 percent protein) was obtained.

In another experiment, soy protein isolate (255 g; ADM 066974, 90% protein) was dispersed at room temperature in sufficient water using a Polytron^{R} Homogenizer (Brinkmann) to obtain a 14 percent dispersion. The dispersion was heated to at 50°C in water bath with agitation. An enzyme blend (3 parts Fungal Protease Concentrate and 1 part Corolase PN-L) was added at 0.5 percent, based on protein weight, and incubated at 50°C. During the first two hours the pH was maintained at about 6.55 to about 6.65 by adding 2N NaOH as necessary. Hydrolysis was continued for an additional half hour without controlling the pH; the pH was about 6.5 after completion of hydrolysis. The enzymes were inactivated in boiling water bath for about 10 to about 13 minutes. After cooling to room temperature, the mixture was centrifuged to obtain a clear supernatant. The supernatant was dried and about 103 g of soluble soy protein (about 40 percent yield with about 77 percent protein) was obtained. Separately, the pellet resulting from the centrifugation was dispersed in water, adjusted to a pH of about 7.0, and freeze-dried to give about 178 g modified soy protein (83% protein). Several similar experiments were carried out using different enzyme contents and hydrolysis times with the following results:

| Enzyme (%) | Incubation Time (hr) | Yield (%) | Protein (%) |
|---|---|---|---|
| 0.5 | 2.5 | 39.0 | 80.4 |
| 0.5 | 2.5 | 40.0 | 77.0 |
| 0.4 | 3.0 | 39.0 | 82.0 |

## Claims

1. A method for preparing a soluble soy protein material, said method comprising:
(1) preparing a hydrolytic mixture comprising water, a soy protein, and an enzyme or a mixture of enzymes having both endo and exo-peptidase activities;
(2) allowing the soy protein to hydrolyze for a sufficient time to produce a soy protein hydrolysate containing at least about 15 percent soluble soy protein; and
(3) deactivating the enzyme or the mixture of enzymes in the soy protein hydrolysate before bitter flavors become noticeable in the soy protein hydrolysate,
wherein the soy protein hydrolysate from step (3) contains the soluble soy protein material.

2. The method of Claim 1, wherein the enzyme or mixture of enzymes comprises a fungal protease enzyme or a mixture of fungal protease enzymes.

3. The method according to Claim 1 or Claim 2, wherein the soy protein hydrolysate from step (3) contains about 15 to about 45 percent the soluble soy protein material.

4. The method according to any one of Claims 1 to 3, wherein the hydrolytic mixture contains about 5 to about 25 percent soy protein and about 0.01 to about 0.5 percent of the fungal protease enzyme or the mixture of fungal protease enzymes.

5. The method according to any one of Claims 2 to 4, wherein the fungal protease enzyme or the mixture of fungal protease enzymes is deactivated at about 80 to about 100°C for about 10 seconds to about 20 minutes.

6. The method according to any one of Claims 1 to 5, wherein the soy protein hydrolysate from step (3) contains the soluble soy protein material and an insoluble soy protein material, wherein the soy protein hydrolysate from step (3) is further processed to separate the soluble soy protein material and insoluble soy protein material.

7. The method according to Claim 6, wherein the soluble soy protein material and the insoluble soy protein material are separated by centrifugation.

8. The method according to Claim 6, wherein the soluble soy protein material and the insoluble soy protein material are separated by filtration.

9. The method according to any one of Claims 1 to 8, wherein the soluble soy protein material comprises about 10 to about 60 percent peptides having a molecular weight of about 3.5 to about 14 kDa, about 20 to 60 percent peptides having a molecular weight of about 14 to about 27 kDa, and about 10 to 40 percent peptides having a molecular weight of greater than about 27 kDa.

10. A method for preparing a soluble soy protein material, said method comprising:
(1) mixing a soy protein with water at about 24 to about 55°C to make a soy paste at a pH of about 6.5 to about 8.0;
(2) adding a fungal protease enzyme or a mixture of fungal protease enzymes having both endo and exo-peptidase activities to the soy paste to form a hydrolytic mixture;
(3) incubating the hydrolytic mixture for at least about 30 minutes at a temperature of about 24 to about 55°C to obtain an incubated hydrolytic mixture containing at least about 15 percent soluble soy protein; and
(4) heating the incubated hydrolytic mixture at a temperature of about 80 to about 100°C for at a time sufficient to inactivate the fungal protease enzyme or the mixture of fungal protease enzymes and to obtain a soy protein hydrolysate, wherein the soy protein hydrolysate contains the soluble soy protein material.

11. The method according to any one of Claims 1 to 10, wherein the soy protein is selected from the group consisting of soy protein isolate, soy protein concentrate, soy protein extract, soy flour, powdered or dry soy milk, soy meal, ground soy bean, soy bean paste, and mixtures thereof.

12. The method according to Claim 10, wherein the incubated hydrolytic mixture contains about 15 to about 45 percent soluble soy protein.

13. The method according to any one of Claims 10 to 12, wherein the soy protein hydrolysate contains the soluble soy protein material and an insoluble soy protein material and wherein the soluble soy protein material and insoluble soy protein material are separated.

14. The method according to any one of Claims 1 to 13, wherein the soluble soy protein material has an antioxidant capacity of total ORAC units per gram of about 50 to about 500.

15. The method according to any one of Claims 1 to 14, wherein the soluble soy protein material is freeze dried or spray-dried.

16. The method according to any one of Claims 1 to 15, wherein the soluble soy protein material comprises peptides having an average molecular weight of about 3 to about 30 kDa.

17. The method according to any one of Claims 1 to 16, wherein the soluble soy protein material contains less than about 10 percent free amino acids.

18. The method according to any one of Claims 1 to 17, wherein the soluble soy protein material contains less than about 7.5 percent free amino acids.

19. The method according to any one of Claims 1 to 18, wherein the soluble soy protein material is soluble in an aqueous medium having a pH of about 2 to about 9.

20. A product comprising a soluble soy protein material, wherein the soluble soy protein material is prepared by a process comprising:
(1) preparing a hydrolytic mixture comprising water, a soy protein, and an enzyme or a mixture of enzymes having both endo and exo-peptidase activities;
(2) allowing the soy protein to hydrolyze for a sufficient time to produce a soy protein hydrolysate containing at least about 15 percent soluble soy protein; and
(3) deactivating the enzyme or the mixture of enzymes in the soy protein hydrolysate before bitter flavors become noticeable in the soy protein hydrolysate,
wherein the soy protein hydrolysate from step (3) contains the soluble soy protein material.

21. The product of Claim 20, wherein the product is a food product, a cosmetic product, or a pharmaceutical product.

22. The product of Claim 20 or 21, wherein the product is a food product.

23. The product according to any one of Claims 20 to 22, wherein the product contains an amount of soluble soy protein material that is recognized to reduce the risk of heart disease, and wherein the amount of soluble soy protein material does not provide a gritty mouthfeel or a bitter taste.

24. The product according to any one of Claims 20 to 23, wherein the soluble soy protein material has an antioxidant capacity of total ORAC units per gram of about 50 to about 500.

25. The product of any one of Claims 21 to 24, wherein the food product is selected from the group consisting of beverages, health/nutrition bars, salad dressings meat products, snacks, desserts, confectionaries, and nutritional supplements.

26. The product according to any one of Claims 21 to 25, wherein the food product is a beverage.

27. A method for preparing a soluble soy protein material, said method comprising:
(1) mixing a soy protein-containing material with water at about 24 to about 55°C to make a soy paste containing about 10 to about 20 percent soy protein at a pH of about 6.5 to about 8.0;
(2) adding about 0.01 to about 0.5 percent of an enzyme or a mixture of enzymes having both endo and exo-peptidase activities to the soy paste to form a hydrolytic mixture;
(3) incubating the hydrolytic mixture for about 0.5 to about 4 hours at a temperature of about 24 to about 55°C to obtain an incubated hydrolytic mixture containing at least about 15 percent soluble soy protein;
(4) heating the incubated hydrolytic mixture at a temperature of about 80 to about 100°C for about 10 sec to about 25 minutes to deactivate the enzyme or the mixture of enzymes and to obtain a soy protein hydrolysate, wherein the soy protein hydrolysate contains the soluble soy protein material and an insoluble/modified soy protein material;
(5) adjusting the pH of the soy protein hydrolysate to about 3.5 to about 5.5;
(6) treating the pH-adjusted soy protein hydrolysate to separate the soluble soy protein material from insoluble/modified soy protein material, wherein the soluble soy protein material is obtained in an amount of at least about 15 to about 45 percent of the soy protein-containing material; and
(7) drying the separated soluble soy protein material to obtain the soluble soy protein material in a solid or powdered form.

28. The method of claim 27, wherein the enzyme or mixture of enzymes comprises a fungal protease enzyme or a mixture of fungal protease enzymes.
